(19) **Europäisches Patentamt / European Patent Office / Office européen des brevets**

(11) **EP 2 921 540 A1**

(12) # EUROPEAN PATENT APPLICATION

(43) Date of publication:
**23.09.2015 Bulletin 2015/39**

(21) Application number: **15159612.9**

(22) Date of filing: **18.03.2015**

(51) Int Cl.:
*C09J 7/00* (2006.01)       *A61F 13/02* (2006.01)
*C08K 5/10* (2006.01)       *C09J 7/02* (2006.01)
*C09J 133/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **18.03.2014   JP 2014054889**

(71) Applicant: **NITTO DENKO CORPORATION**
**Osaka (JP)**

(72) Inventors:
• **Nakagawa, Masao**
  **Ibaraki-shi, Osaka (JP)**
• **Kikuta, Noriyuki**
  **Ibaraki-shi, Osaka (JP)**
• **Hamada, Atsushi**
  **Ibaraki-shi, Osaka (JP)**
• **Higashio, Kazuhiro**
  **Ibaraki-shi, Osaka (JP)**

(74) Representative: **Grünecker Patent- und Rechtsanwälte**
**PartG mbB**
**Leopoldstraße 4**
**80802 München (DE)**

(54) **PRESSURE-SENSITIVE ADHESIVE TAPE OR SHEET FOR APPLICATION ON SKIN**

(57)     A pressure-sensitive adhesive tape or sheet for application on skin includes a substrate and a pressure-sensitive adhesive layer laminated on at least one surface of the substrate. The pressure-sensitive adhesive layer contains: 100 parts by weight of an acrylic copolymer prepared by copolymerizing a monomer mixture comprising from 5 to 38% by weight of an alkyl (meth) acrylate monomer, from 61 to 90% by weight of an alkoxy group-containing ethylenic unsaturated monomer and from 1 to 10% by weight of a carboxyl group-containing ethylenic unsaturated monomer; and from 30 to 60 parts by weight of a carboxylic acid ester in a paste form or liquid form at room temperature. The acrylic copolymer has been subjected to a crosslinking treatment.

EP 2 921 540 A1

**Description**

BACKGROUND OF THE INVENTION

Technical Field

The present disclosure relates to a pressure-sensitive adhesive tape or sheet for application on skin for use in the medical and healthcare field.

Background Art

In general, the pressure-sensitive adhesive for use in a pressure-sensitive adhesive tape or sheet for application on skin must surely bond to the surface of skin and therefore the skin adhesion force must be set to be high in some degree. However, when the skin adhesion force is set to be high, then there is a probability that skin irritation is caused when the pressure-sensitive adhesive tape or sheet is peeled off from the skin surface. In addition, when the pressure-sensitive adhesive tape or sheet is kept laminated to the surface of skin for a long period of time, then the skin surface may be itchy or, various dermatitides may be caused in some cases.

In addition, in general, the surface of skin has an irregular and complicated structure, and therefore the pressure-sensitive adhesive is difficult to completely adhere to the skin surface to be applied. Consequently, it is considered to improve the adhesiveness by softening the pressure-sensitive adhesive. However, when the pressure-sensitive adhesive is softened, the cohesion force of the pressure-sensitive adhesive is lowered, and consequently, the softened pressure-sensitive adhesive may remain on the skin surface when the pressure-sensitive adhesive tape or sheet for application on skin is peeled off from the skin surface, and therefore, such a case may cause adhesive deposits. To that effect, the pressure-sensitive adhesive to be laminated to the surface of skin is required to satisfy a delicate balance between the internal cohesion force and the skin adhesion force of the pressure-sensitive adhesive layer (see Patent Document 1).

Further, when a pressure-sensitive adhesive tape or sheet for application on skin is laminated to the surface of skin, the pressure-sensitive adhesive tape or sheet gets easily stuffy, which would cause skin irritation, and therefore those having a moisture permeability as high as possible are desired. However, the moisture permeability of already-existing pressure-sensitive adhesive tape or sheet for application on skin could hardly be said as yet to be on a sufficient level.

Patent Document 1: JP-A 2004-097730

SUMMARY OF THE INVENTION

In consideration of the current situation as mentioned above, an object of the present disclosure is to provide a pressure-sensitive adhesive tape or sheet for application on skin which has good adhesion to the surface of skin, can reduce skin irritation when peeled off from the surface of skin and has excellent moisture permeability.

The present inventors have assiduously studied for the purpose of attaining the above-mentioned object and, as a result, have found that, in a pressure-sensitive adhesive tape or sheet for application on skin, when the pressure-sensitive adhesive layer contains an acrylic copolymer formed from a specific monomer composition and a carboxylic acid ester, in which the acrylic copolymer has been subjected to crosslinking treatment, then flexibility and cohesive property are given to the pressure-sensitive adhesive layer and, in addition, high moisture permeability as compared with that in already-existing cases can be given to the pressure-sensitive adhesive layer, and thus, the present invention has been completed.

The present invention provides the following pressure-sensitive adhesive tape or sheet for application on skin.

(1) A pressure-sensitive adhesive tape or sheet for application on skin, comprising a substrate and a pressure-sensitive adhesive layer laminated on at least one surface of the substrate,
wherein the pressure-sensitive adhesive layer comprises: 100 parts by weight of an acrylic copolymer prepared by copolymerizing a monomer mixture comprising from 5 to 38% by weight of an alkyl (meth)acrylate monomer, from 61 to 90% by weight of an alkoxy group-containing ethylenic unsaturated monomer and from 1 to 10% by weight of a carboxyl group-containing ethylenic unsaturated monomer; and from 30 to 60 parts by weight of a carboxylic acid ester in a paste form or liquid form at room temperature, and the acrylic copolymer has been subjected to a crosslinking treatment.

(2) The pressure-sensitive adhesive tape or sheet for application on skin according to (1), wherein the carboxylic acid ester is a glycerin ester of a saturated fatty acid.

(3) The pressure-sensitive adhesive tape or sheet for application on skin according to (2), wherein the saturated fatty acid is at least one selected from the group consisting of caprylic acid, capric acid and 2-ethylhexanoic acid.
(4) The pressure-sensitive adhesive tape or sheet for application on skin according to any one of (1) to (3), wherein the carboxylic acid ester is at least one selected from the group consisting of triglyceryl caprylate, triglyceryl caprate, and triglyceryl 2-ethylhexanoate.

According to the present disclosure, the pressure-sensitive adhesive tape or sheet for application on skin having excellent adhesion property to the surface of skin and capable of reducing skin irritation when peeled off from the surface of skin can be provided. In addition, the pressure-sensitive adhesive tape or sheet has high moisture permeability, and can prevent the skin surface from getting stuffy and being itchy while kept laminated to the surface of skin, and thus, the pressure-sensitive adhesive tape or sheet is therefore useful in long-term lamination thereof on skin.

DETAILED DESCRIPTION OF THE INVENTION

Preferred embodiments of the present invention are described below; however, the present invention is not limited to these specific embodiments.

In the present disclosure, the pressure-sensitive adhesive layer constituting the pressure-sensitive adhesive tape or sheet for application on skin contains: 100 parts by weight of an acrylic copolymer prepared by copolymerizing a monomer mixture containing from 5 to 38% by weight of an alkyl (meth)acrylate monomer, from 61 to 90% by weight of an alkoxy group-containing ethylenic unsaturated monomer and from 1 to 10% by weight of a carboxyl group-containing ethylenic unsaturated monomer; and from 30 to 60 parts by weight of a carboxylic acid ester in a paste form or liquid form at room temperature, and the acrylic copolymer has been subjected to a crosslinking treatment.

The alkyl (meth)acrylate to be used for preparing the acrylic copolymer in the present disclosure is a component that gives pressure-sensitive adhesive property and skin adhesiveness to the pressure-sensitive adhesive layer, and in particular, use of a long-chain alkyl ester where the alkyl group has 6 or more carbon atoms, preferably from 6 to 18 carbon atoms, is effective. In addition, the following advantages can be achieved based on the use of the alkyl (meth)acrylate monomer: as compared with a rubber-based pressure-sensitive adhesive, the copolymer is contaminated with few impurities and is relatively less irritative to skin and the pressure-sensitive adhesive property thereof hardly lowers in long-term use.

Specific examples of the alkyl (meth)acrylate monomer include butyl (meth)acrylate, propyl (meth)acrylate, octyl (meth)acrylate, nonyl (meth)acrylate, decyl (meth)acrylate, dodecyl (meth)acrylate and lauryl (meth)acrylate, and one or more of these may be used here either singly or as combined. The alkyl ester chains may be linear or branched ones.

The alkyl (meth)acrylate monomer is copolymerized with an unsaturated monomer to be mentioned below, thereby forming the acrylic copolymer for use in the present disclosure. The alkyl (meth)acrylate monomer is contained, in the monomer component to be polymerized, in a ratio of from 5 to 38% by weight, preferably from 15 to 30% by weight. When the content of the alkyl (meth)acrylate monomer is from 5 to 38% by weight, good pressure-sensitive adhesive property to the surface of skin can be secured.

The alkoxy group-containing ethylenic unsaturated monomer to be copolymerized with the alkyl (meth)acrylate monomer is a component that gives water vapor permeability to the resultant copolymer. As the unsaturated monomer, preferable examples thereof include an alkoxyalkyl acrylate in which the alkoxy group has from 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate and butoxyethyl acrylate. The alkoxy group-containing ethylenic unsaturated monomer is contained, in the monomer component to be polymerized, in a ratio of from 61 to 90% by weight, preferably from 65 to 80% by weight. When the content of the alkoxy group-containing ethylenic unsaturated monomer is from 61 to 90% by weight, excellent moisture permeability can be secured and it is effective for preventing from getting stuffy or being itchy during the lamination of the pressure-sensitive adhesive tape or sheet to the surface of the skin.

In the present disclosure, in addition to the alkoxy group-containing unsaturated monomer, a carboxyl group-containing ethylenic unsaturated monomer is further copolymerized to improve the cohesion force of the resultant copolymer. However, when a large quantity of the monomer is used for the copolymerization, improvement of the internal cohesion force can be expected but skin irritation would be gradually increased. Consequently, the carboxyl group-containing ethylenic unsaturated monomer is contained, in the monomer component to be polymerized, in a ratio of from 1 to 10% by weight, preferably from 3 to 7% by weight. Typical examples of the carboxyl group-containing ethylenic unsaturated monomer include acrylic acid, methacrylic acid, itaconic acid, crotonic acid, fumaric acid, maleic acid, maleic anhydride and the like. Of those, acrylic acid and methacrylic acid are preferred as the carboxyl group-containing ethylenic unsaturated monomer from the viewpoint of the copolymerizability and handleability thereof.

As the acrylic copolymer to be contained in the pressure-sensitive adhesive in the present disclosure, copolymers of the above-mentioned monomers may be used. If desired, as a modifying monomer for various modifications such as hydrophilization, the monomer such as styrene, vinyl acetate or N-vinyl-2-pyrrolidone may be further copolymerized.

The acrylic copolymer in the present disclosure may be prepared according to a conventional polymerization method such as a solution polymerization method, an emulsion polymerization method or a suspension polymerization. The copolymer may also be prepared through radical polymerization using a radical polymerization initiator such as a peroxide compound or an azo-based compound.

Preferably, the acrylic copolymer has a glass transition temperature of 250°K or lower. In other words, the copolymer having a glass transition temperature of 250°K or lower secures sufficient skin adhesiveness of the pressure-sensitive adhesive tape or sheet for application on skin when the pressure-sensitive adhesive tape or sheet is kept laminated to the surface of skin. Also preferably, the weight-average molecular weight of the acrylic copolymer is controlled to be 1,000,000 or less, more preferably from about 500,000 to 900,000.

The glass transition temperature and the weight-average molecular weight of the acrylic copolymer may be controlled by suitably controlling the polymerization conditions (monomer species, monomer blending ratio, solid content ratio, reaction temperature, reaction time, etc.) for the acrylic copolymer.

The pressure-sensitive adhesive layer in the present disclosure contains the above-mentioned acrylic copolymer and additionally a carboxylic acid ester that is a paste form or liquid form at room temperature, as the indispensable components therein. The carboxylic acid ester to be contained in the pressure-sensitive adhesive layer along with the above-mentioned acrylic copolymer therein is in a paste form or liquid form at room temperature (25°C), and this provides characteristics of lowering the modulus in the low-deformation region of the pressure-sensitive adhesive, keeping good adhesiveness to the surface of skin, and reducing corneum damage and pain when the pressure-sensitive adhesive tape or sheet is peeled. The carboxylic acid ester in a solid form at room temperature is unfavorable since, when mixed with the acrylic copolymer, it could hardly disperse or dissolve uniformly and may therefore cause nonuniform adhesion properties.

The carboxylic acid ester has affinity to the acrylic copolymer and is miscible with the acrylic copolymer, and specific examples thereof include carboxylic acid esters with a monohydric alcohol such as ethyl myristate, isopropyl myristate, isopropyl palmitate, butyl stearate, isopropyl isostearate, hexyl laurate, cetyl lactate, myristyl lactate, diethyl phthalate, octyldodecyl myristate, octyldodecyl oleate, hexyldecyl dimethyloctanoate, decyl 2-ehtylhexanoate, isocetyl 2-ethylhexanoate, stearyl 2-ethylhexanoate and dioctyl succinate; carboxylic acid esters with a di- or more polyalcohol such as propylene glycol dicaprylate, propylene glycol dicaprate, propylene glycol diisostearates, glyceryl monocaprylate, glyceryl tricaprylate, glyceryl tri-2-ehtylhexanoate, glyceryl tricaprate, glyceryl trilaurate, glyceryl triisostearate, glyceryl trioleate and trimethylolpropane tri-2-ehtylhexanoate.

Of these carboxylic acid esters, as those which is less subject to oxidative degradation, use of an ester of glycerin and a saturated fatty acid not having an unsaturated bond therein is preferable from the viewpoint of the quality stability thereof. Preferred examples of the saturated fatty acid include caprylic acid, capric acid, 2-ethylhexanoic acid and the like. Especially preferred examples thereof include triglyceryl caprylate, triglyceryl caprate, triglyceryl 2-ehtylhexanoate and the like.

One kind of the carboxylic acid esters or two or more of the carboxylic acid esters as combined is/are components to be mixed with the acrylic copolymer to form the pressure-sensitive adhesive layer. The content of the carboxylic acid ester in the pressure-sensitive adhesive layer is controlled to fall within a range of from 30 to 60 parts by weight, preferably from 35 to 55 parts by weight relative to 100 parts by weight of the acrylic copolymer. In case where the content of the carboxylic acid ester is less than 30 parts by weight, when the pressure-sensitive adhesive layer of the pressure-sensitive adhesive tape or sheet for application on skin in the present disclosure is laminated to the surface of skin, the pressure-sensitive adhesive layer could not sufficiently absorb the sweat from the skin surface and therefore the skin adhesiveness thereof would lower. On the other hand, when the content of the carboxylic acid ester is more than 60 parts by weight, the pressure-sensitive adhesive layer could be excessively plasticized and the skin adhesion force thereof would greatly lower.

The pressure-sensitive adhesive layer in the pressure-sensitive adhesive tape or sheet for application on skin of the present invention contains the acrylic copolymer and the carboxylic acid ester as the indispensable components therein, but within a range not overstepping the object of the present invention and if desired, various additives such as conventional plasticizer, softener, filler, tackifier, antimicrobial agent or microbicide may be suitably added thereto.

In the present disclosure, the uncrosslinked acrylic copolymer is subjected to a crosslinking treatment. By subjecting the acrylic copolymer to the crosslinking treatment, the cohesion force of the pressure-sensitive adhesive layer can be enhanced and good skin adhesiveness can be expressed.

Examples of the crosslinking treatments include physical crosslinking treatment through γ-ray irradiation, electron beam irradiation or the like, and chemical crosslinking treatment with an organic peroxide, an isocyanate compound, an organic metal salt, a metal alcoholate, a metal chelate compound, an epoxy compound, a primary amino group-containing compound or the like. Specific examples for the chemical crosslinking treatment include organic peroxides such as benzoyl peroxide; isocyanate compounds such as tolylene diisocyanate and hexamethylene diisocyanate; epoxy compounds such as glycerin triglycidyl ether and triglycidyl isocyanurate; metal chelate compounds such as aluminium tris(acetylacetonate) and ethylacetoacetate aluminium diisopropylate. Of those, preferred examples thereof include isocyanate compounds, metal alcoholates and metal chelate compounds from the viewpoint of the crosslinking reactivity and of the easiness in handling them. By such a crosslinking treatment, the balance between the flexibility and the cohesion force of the pressure-sensitive adhesive layer can be suitably controlled and the pressure-sensitive adhesive layer components can be surely held, and therefore, bleeding of the pressure-sensitive adhesive layer components and adhesive deposits can be prevented.

The thickness of the pressure-sensitive adhesive layer is preferably from about 10 to 60 $\mu$m. When the thickness is less than 10 $\mu$m, sufficient skin adhesiveness when laminated to skin may not be exhibited; and when the thickness is more than 60 $\mu$m, sufficient water vapor permeability may not be secured as a whole of the pressure-sensitive adhesive sheet and skin irritation may be caused in long-term lamination.

Not specifically defined, the substrate for use in the present disclosure may be any ones having high mechanical strength not detracting from the flexibility of the pressure-sensitive adhesive tape or sheet laminated to skin, and various structures formed of various materials can be used here. Regarding the structures, examples thereof include fiber aggregates of woven fabric, nonwoven fabric, knitted fabric, paper or the like; and sheets such as films such as porous film or vapor-permeable film, and the like. Of those, especially preferred are ones having suitable flexibility that enables the pressure-sensitive adhesive tape or sheet to follow the curved surface to which it is laminated. The substrate may be a sheet-form single layer or may be a sheet-form laminate. Examples of materials of the substrate include polyurethane polymers such as polyether urethane and polyester urethane; amide polymers such as polyether polyamide block polymer; acrylic polymers such as polyacrylate; polyolefin polymers such as polyethylene, polypropylene and ethylene/vinyl acetate copolymer; polyester polymers such as polyether polyester. Preferably, the substrate is selected from a fabric such as nonwoven fabric, or a polymer of a material having water-vapor permeability, for preventing the skin surface to which the pressure-sensitive adhesive tape or sheet is laminated from getting stuffy or being whitened. For example, use of an urethane-based or amide-based sheet material is preferable.

The substrate may be perforated from the viewpoint of preventing from getting stuffy or being itchy during the lamination of the pressure-sensitive adhesive tape or sheet. Perforation of the substrate may be easily attained according to a conventional perforation technique based on a metal roll, a perforator or laser irradiation. As a method of perforating the substrate in the pressure-sensitive adhesive tape or sheet for application on skin in the present disclosure, the substrate itself may be first perforated and then a pressure-sensitive adhesive layer may be laminated thereto; or a pressure-sensitive adhesive layer may be first laminated to the substrate and then the resultant substrate may be then perforated. In the case where a pressure-sensitive adhesive layer is first laminated to a substrate and then the substrate is perforated, the formed perforations run through both the substrate and the pressure-sensitive adhesive layer.

It is desirable that the thickness of the substrate is suitably determined depending on the material, the use thereof or the like, but in general, when a film is used as the substrate, its thickness is preferably from 3 to 30 $\mu$m, more preferably from 5 to 20 $\mu$m. When a fabric such as a woven fabric or a nonwoven fabric, or paper or the like is used as the substrate, it may be difficult to measure the absolute thickness thereof in some cases. In the case, the weight of the substrate is preferably from 10 to 100 g/m$^2$, more preferably from 20 to 60 g/m$^2$.

In general, on an exposed surface of the pressure-sensitive adhesive layer of the pressure-sensitive adhesive tape or sheet for application on skin in the present disclosure, a release sheet the surface of which has been subjected to release treatment such as silicone treatment is temporarily laminated.

Example

The present invention is described concretely with reference to the following Examples, but these Examples are not to restrict the scope of the present invention.

(Example 1)

A monomer mixture containing 30 parts of isononyl acrylate, 65 parts of 2-methoxyethyl acrylate and 5 parts of acrylic acid was uniformly dissolved and mixed in 100 parts of toluene, and 0.2 parts of azobisisobutyronitrile as a polymerization initiator was added thereto, thereby carrying out copolymerization reaction.

Based on 100 parts of the resultant acrylic copolymer, 60 parts of triglyceryl caprylate and 0.13 parts of a trifunctional isocyanate compound (trade name "CORONATE HL" manufactured by Nippon Polyurethane Industry Co., Ltd.) were mixed in toluene, thereby giving a uniform pressure-sensitive adhesive solution.

Next, the solution was applied onto the release-treated surface of a release sheet that had been release-treated in such a manner that the dry thickness could be 15 $\mu$m, and then dried at 120°C for 3 minutes, thereby forming a pressure-sensitive adhesive layer. Next, the formed pressure-sensitive adhesive layer was transferred and laminated to one surface of a polyether polyurethane film (thickness 15 $\mu$m), and then ripened at 60°C for 72 hours, thereby producing a pressure-sensitive adhesive tape for application on skin in the present disclosure.

(Example 2)

A pressure-sensitive adhesive tape for application on skin in the present disclosure was produced in the same manner as in Example 1 except that 45 parts of triglyceryl caprylate was used.

(Example 3)

A pressure-sensitive adhesive tape for application on skin in the present disclosure was produced in the same manner as in Example 1 except that 30 parts of triglyceryl caprylate was used.

(Comparative Example 1)

A pressure-sensitive adhesive tape for application on skin was produced in the same manner as in Example 1 except that triglyceryl caprylate was not added.

(Comparative Example 2)

A pressure-sensitive adhesive tape for application on skin was produced in the same manner as in Example 1 except that an acrylic copolymer prepared through copolymerization of a monomer mixture containing 65 parts of isononyl acrylate, 30 parts of 2-methoxyethyl acrylate and 5 parts of acrylic acid and uniformly dissolved and mixed in 40 parts of toluene was used.

The pressure-sensitive adhesive tapes for application on skin produced in each of Examples and Comparative Examples were tested as mentioned below, and the results are shown in Table 1.

(Skin pressure-sensitive adhesive force)

The pressure-sensitive adhesive sheet that had been cut to have a rectangular form of 20 mm in width x 60 mm in length was laminated to the forearm of 4 volunteers, by pressing it with a 1-kg roller moved back and forth once. After the lapse of time of 6 hours at room temperature in this condition, the pressure-sensitive adhesive sheet was peeled in the 180-degree direction at a peeling speed of 300 mm/min, and the peeling force was measured at this time. The data of the peeling force of the 4 volunteers were averaged and the resultant mean value is referred to as the value of the skin pressure-sensitive adhesive force of the tested sample.

(Skin irritation (pain) when peeling)

In the above-mentioned test for the skin pressure-sensitive adhesive force, the degree of skin irritation (pain), when the pressure-sensitive adhesive sheet was peeled, was measured and evaluated in 5 ranks. The highest score 5 means no skin irritation, and the lowest score 1 means severe skin irritation. The data of the 4 volunteers were averaged to calculate the mean value. The smaller scores indicate severer skin irritation (pain).

(Moisture Permeation Test)

10 ml of distilled water was put in a glass vessel (weighing bottle) having an inner diameter of 40 mm and a height of 40 mm, and the pressure-sensitive adhesive tape for application on skin cut into a disc shape having a diameter of 50 mm was laminated to the mouth of the vessel in a state where the pressure-sensitive adhesive layer thereof faced downward, and was fixed. The weight (W1) of the entire vessel to which the pressure-sensitive adhesive tape had been fixed was measured. Subsequently, this was put into a constant-temperature constant moisture chamber having a temperature of 40°C and a relative humidity of 30% R.H., and after kept therein for 24 hours, the weight thereof (W2) was measured. The moisture permeability was calculated according to the following formula. The same test was repeated 3 times for every pressure-sensitive adhesive tape for application on skin produced in each of Examples and Comparative Examples, and the mean value of each sample was calculated.

$$\text{Moisture Permeability (g/m}^2\cdot\text{24h)} = (W1 - W2)/(0.02 \times 0.02 \times \pi)$$

The moisture permeability of the pressure-sensitive adhesive tape or sheet for application on skin as a whole in the present disclosure is preferably 2500 g/m²·24h or more. In a case of laminating the pressure-sensitive adhesive tape or sheet to the surface of a human skin, it is desirable that the pressure-sensitive adhesive tape or sheet has a moisture permeability of 2500 g/m$^2$·24h or more, though varying depending on the individual difference and on the application site. This is because the pressure-sensitive adhesive tape or sheet having the moisture permeability on this level could exhibit sufficient moisture permeation even though the pressure-sensitive adhesive tape or sheet is laminated to a site that may produce much sweat, and therefore, the pressure-sensitive adhesive tape or sheet could prevent the skin surface from getting stuffy or being whitened.

[Table 1]

|  | Skin pressure-sensitive adhesive force (N/20 mm width) | Skin irritation (pain) | Moisture permeability (g/m$^2$·24h) |
|---|---|---|---|
| Example 1 | 0.31 | 4.3 | 3053 |
| Example 2 | 0.25 | 4.3 | 2967 |
| Example 3 | 0.26 | 4.0 | 2781 |
| Comparative Example 1 | 0.98 | 3.0 | 2582 |
| Comparative Example 2 | 0.74 | 3.0 | 2105 |

As obvious from the results in Table 1, the pressure-sensitive adhesive tapes or sheets in Examples had a high-level moisture permeability and were effective for preventing the skin surface from getting stuffy and being whitened. In addition, in peeling the pressure-sensitive adhesive tape or sheet, the pressure-sensitive adhesive force was lowered and the skin irritation (pain) was low, that is, these pressure-sensitive adhesive tapes or sheets had good peelability. On the other hand, the pressure-sensitive adhesive force in peeling the pressure-sensitive adhesive tapes or sheets in Comparative Examples was all high, and in peeling them, users would suffer from skin irritation (pain). In addition, the moisture permeability of the pressure-sensitive adhesive tape or sheet in Comparative Example 2 was relatively low, and therefore, when the pressure-sensitive adhesive tape or sheet is used in a steamy summer, or when the pressure-sensitive adhesive tape or sheet is laminated to the site that may produce much sweat, the skin surface would get stuffy or be whitened.

While the invention has been described in detail and with reference to specific embodiments thereof, it will be apparent to one skilled in the art that various changes and modifications can be made therein without departing from the spirit and scope thereof.

This application is based on Japanese Patent Application No. 2014-054889 filed on March 18, 2014, the entire subject matter of which is incorporated herein by reference.

**Claims**

1. A pressure-sensitive adhesive tape or sheet for application on skin, comprising a substrate and a pressure-sensitive adhesive layer laminated on at least one surface of the substrate,
wherein the pressure-sensitive adhesive layer comprises: 100 parts by weight of an acrylic copolymer prepared by copolymerizing a monomer mixture comprising from 5 to 38% by weight of an alkyl (meth)acrylate monomer, from 61 to 90% by weight of an alkoxy group-containing ethylenic unsaturated monomer and from 1 to 10% by weight of a carboxyl group-containing ethylenic unsaturated monomer; and from 30 to 60 parts by weight of a carboxylic acid ester in a paste form or liquid form at room temperature, and the acrylic copolymer has been subjected to a crosslinking treatment.

2. The pressure-sensitive adhesive tape or sheet for application on skin according to claim 1, wherein the carboxylic acid ester is a glycerin ester of a saturated fatty acid.

3. The pressure-sensitive adhesive tape or sheet for application on skin according to claim 2, wherein the saturated fatty acid is at least one selected from the group consisting of caprylic acid, capric acid and 2-ethylhexanoic acid.

4. The pressure-sensitive adhesive tape or sheet for application on skin according to any one of claims 1 to 3, wherein the carboxylic acid ester is at least one selected from the group consisting of triglyceryl caprylate, triglyceryl caprate, and triglyceryl 2-ethylhexanoate.

Europäisches
Patentamt

European
Patent Office

Office européen
des brevets

# EUROPEAN SEARCH REPORT

Application Number

EP 15 15 9612

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2005 304756 A (NITTO DENKO CORP) 4 November 2005 (2005-11-04) * paragraphs [0044] - [0050]; claim 1 * ----- | 1-4 | INV. C09J7/00 A61F13/02 C08K5/10 C09J7/02 C09J133/00 |
| A | US 2002/061948 A1 (MURAKAMI YOSHIHIDE [JP] ET AL) 23 May 2002 (2002-05-23) * paragraphs [0008], [0015], [0018]; examples 1,5; table 1 * ----- | 1-4 | |
| A | EP 1 060 719 A1 (NICHIBAN KK [JP]) 20 December 2000 (2000-12-20) * paragraphs [0032] - [0033] * ----- | 1 | |

TECHNICAL FIELDS
SEARCHED      (IPC)

C09J
A61F
C08K

The present search report has been drawn up for all claims

| Place of search | Date of completion of the search | Examiner |
|---|---|---|
| Munich | 7 August 2015 | Alevizopoulou, M |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

................................................................

& : member of the same patent family, corresponding
     document

EPO FORM 1503 03.82 (P04C01)

**ANNEX TO THE EUROPEAN SEARCH REPORT
ON EUROPEAN PATENT APPLICATION NO.**                     EP 15 15 9612

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2015

| Patent document cited in search report | | Publication date | Patent family member(s) | | Publication date |
|---|---|---|---|---|---|
| JP 2005304756 | A | 04-11-2005 | NONE | | |
| US 2002061948 | A1 | 23-05-2002 | AT | 307178 T | 15-11-2005 |
| | | | CA | 2356468 A1 | 01-03-2002 |
| | | | DE | 60114108 T2 | 20-07-2006 |
| | | | DK | 1184039 T3 | 07-11-2005 |
| | | | EP | 1184039 A2 | 06-03-2002 |
| | | | ES | 2246971 T3 | 01-03-2006 |
| | | | KR | 20020018593 A | 08-03-2002 |
| | | | US | 2002061948 A1 | 23-05-2002 |
| EP 1060719 | A1 | 20-12-2000 | AT | 265836 T | 15-05-2004 |
| | | | DE | 69917009 D1 | 09-06-2004 |
| | | | DE | 69917009 T2 | 14-04-2005 |
| | | | DK | 1060719 T3 | 30-08-2004 |
| | | | EP | 1060719 A1 | 20-12-2000 |
| | | | JP | 2000189453 A | 11-07-2000 |
| | | | US | 6297421 B1 | 02-10-2001 |
| | | | WO | 0040189 A1 | 13-07-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004097730 A

- JP 2014054889 A